# EUROPEAN PATENT SPECIFICATION

(11) **EP 4 380 474 B1**
(45) Date of publication and mention of the grant of the patent: **28.10.2026**
(21) Application number: 22757363.1
(22) Date of filing: 02.08.2022
(51) Int. Cl.: A61B 17/22, A61M 1/00, A61M 39/24, A61M 39/22

(54) **PASSIVE FLOW SWITCH FOR MEDICAL ASPIRATION**
PASSIVER STRÖMUNGSSCHALTER FÜR MEDIZINISCHE ASPIRATION
COMMUTATEUR D'ÉCOULEMENT PASSIF POUR ASPIRATION MÉDICALE

(30) Priority: 02.08.2021 US 202163228364 P; 27.06.2022 US 202217809118
(43) Date of publication of application: 12.06.2024
(73) Proprietor: Covidien LP, Mansfield, MA 02048 (US)
(72) Inventor: WOOD, Rónán, Ballybrit, Galway (IE)
(74) Representative: Zimmermann & Partner Patentanwälte mbB
(86) International application number: PCT/IB2022/057167
(87) International publication number: WO 2023/012665

(56) References cited:
- US-A- 2 121 936
- US-A- 3 085 589
- US-A- 4 030 520
- US-A1- 2002 092 566
- US-A1- 2009 032 121
- US-A1- 2017 014 617
- US-A1- 2021 220 528
- US-B1- 6 579 263

## Description

### TECHNICAL FIELD

This disclosure relates to medical aspiration.

### BACKGROUND

In some cases, medical aspiration can be used to remove material from a patient. For example, medical aspiration can be used to remove a thrombus, such as a clot or other occlusion, from a blood vessel of a patient. Document US 2009/032121 A1 relates to an injection system configured to prevent backflow. Document US 2017/014617 A1 relates to a drainage system. Document US 2021/0220528 A1 discloses an aspiration system including a flow switch.

### SUMMARY

The invention is defined by the appended claims and relates to a flow switch and a medical aspiration system. Methods described herein are useful to understand the invention. This disclosure describes example devices and systems configured to reduce or prevent a withdrawal of body fluid (e.g., blood) from a patient during a medical aspiration procedure, and related methods. An aspiration catheter can be used to remove a thrombus from a hollow anatomical structure (e.g., a blood vessel) of a patient. For example, a distal opening of the catheter may be positioned in the hollow anatomical structure near a thrombus and an aspiration force can be applied to a lumen of the aspiration catheter in order to draw the thrombus through the catheter lumen and out of the hollow anatomical structure. In examples described herein, an aspiration system includes an aspiration catheter fluidically coupled to a flow switch (also referred to herein as a valve) configured to passively (e.g., without human intervention) close in response a presence of an aspirated fluid flow through the valve, in order to reduce or prevent further fluid flow through the aspiration catheter from the hollow anatomical structure. In some examples, the valve includes a spring-suspended plug configured to close the valve in response to an above-threshold drag force applied by the fluid flow onto the plug. Upon the sufficient reduction or total absence of the fluid flow (e.g., when an opening of the catheter engages a more-solid material, such as a thrombus), the valve is configured to automatically return to an "open" position, enabling aspiration of the more-solid material through the catheter.

The devices, systems, and techniques of this disclosure may provide one or more advantages and benefits. For instance, by passively closing the switch in the presence of a fluid flow, the systems and techniques described herein can help reduce the undesired and/or unnecessary withdrawal of a body fluid, such as blood, from a body of a patient during an aspiration procedure, while still allowing for effective removal of targeted material. This more-precise targeted aspiration may help improve patient outcomes. Additionally, the passive nature of the flow switch enables the switch to rapidly close with no required human intervention, enabling the clinician to focus on other aspects of the aspiration procedure. Additionally, the passive flow switches described herein do not require any power source or electronic control, enabling the flow switches to operate without external electrical connections, thereby preserving the sterile field during the medical procedure.

In some examples, a flow switch includes a housing defining an internal cavity and proximal and distal openings to the internal cavity, wherein the internal cavity is configured to receive an aspirated fluid flow from a catheter; and a plug disposed within the internal cavity and configured to: move proximally, in response to an above-threshold drag force from the fluid flow applied to a distal-facing surface of the plug, to close the proximal opening; and move distally to open the proximal opening in response to an absence of the fluid flow within the internal cavity.

In some examples, a medical aspiration system includes a suction source; aspiration tubing fluidically coupled to the suction source, wherein the aspiration tubing defines an inner lumen; and a flow switch fluidically coupled to the aspiration tubing, the flow switch comprising: a housing defining an internal cavity configured to receive an aspirated fluid flow from a catheter; and a proximal opening to the internal cavity, the proximal opening configured to fluidically connect to a distal end of the aspiration tubing; and a distal opening to the internal cavity, the distal opening configured to fluidically connect to the catheter; and a plug disposed within the internal cavity and configured to: move proximally, in response to an above-threshold drag force from the fluid flow applied to a distal-facing surface of the plug, to close the proximal opening; and move distally to open the proximal opening in response to an absence of the fluid flow within the internal cavity.

This disclosure also describes examples of methods of using the aspiration systems and devices. For instance, in some examples, a method includes fluidically coupling a proximal opening to an internal cavity of a flow switch to a distal end of aspiration tubing coupled to a suction source; fluidically coupling a distal opening to the internal cavity of the flow switch to a proximal end of an aspiration catheter, wherein the flow switch further comprises a plug disposed within the internal cavity and configured to: move proximally, in response to an above-threshold drag force from the fluid flow applied to a distal-facing surface of the plug, to close the proximal opening; and move distally to open the proximal opening in response to an absence of the fluid flow within the internal cavity; introducing a distal portion of the catheter into vasculature of a patient; aspirating a thrombus from the vasculature of the patient via the catheter; and withdrawing the catheter from the vasculature of the patient.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

FIG. 1 is a schematic diagram illustrating an example aspiration system including a passive flow switch.
FIG. 2A is a cross-sectional view of an example of the flow switch of FIG. 1 in an "open" configuration.
FIG. 2B is a cross-sectional view of the flow switch of FIG. 2A in a "closed" configuration.
FIG. 3 is a cross-sectional view of the flow switch of FIGS. 2A and 2B.
FIG. 4A is a cross-sectional view of another example of the flow switch of FIG. 1.
FIG. 4B is another cross-sectional view of the flow switch of FIG. 4A.
FIG. 5 is a perspective view of an example proximal housing portion of the flow switch of FIGS. 4A and 4B.
FIG. 6A is a side view of an example plug of the flow switch of FIGS. 4A and 4B.
FIG. 6B is an end view of the example plug of FIG. 6A.
FIG. 7 is a flow diagram of an example method of using an aspiration system.

### DETAILED DESCRIPTION

This disclosure describes devices and systems configured to passively disrupt or inhibit a flow of a body fluid, such as blood, from a body of a patient during a medical aspiration procedure, as well as medical aspiration systems (e.g., vascular aspiration systems) including such devices and systems, and corresponding methods. In examples described herein, an aspiration system includes a flow switch (also referred to herein as a "valve") configured to passively (e.g., without further human intervention) close a fluid pathway from a body of a patient to outside the patient (e.g., a discharge reservoir configured to collect aspirated material) in response to the presence of an above-threshold fluid flow aspirated into a catheter and through the valve. When the fluid flow is sufficiently reduced or is no longer present, such as when a distal opening of the catheter is in contact with a more-solid thrombus material, the valve is configured to passively re-open the fluid pathway such that the thrombus material may be removed from the patient through the catheter lumen.

In some examples, the passive flow switch includes a plug suspended between opposing proximal and distal springs. While a body fluid is aspirated proximally through the catheter, an above-threshold drag force imparted (e.g., from the above-threshold fluid flow) onto the plug causes the plug to move proximally and seal a proximal opening of the switch, thereby modifying (e.g., restricting or preventing) the flow of fluid through the switch. In this way, the flow switch helps reduce an undesired and unnecessary aspiration removal of fluid, thereby improving medical aspiration procedures. Additionally, the passive nature of the flow switch enables the switch to rapidly close with no required human intervention, enabling the clinician to focus on other aspects of the aspiration procedure. Additionally, the passive flow switches described herein do not require any power source or electronic control circuitry, enabling the flow switches to operate without external electrical connections, thereby preserving the sterile field during the medical procedure.

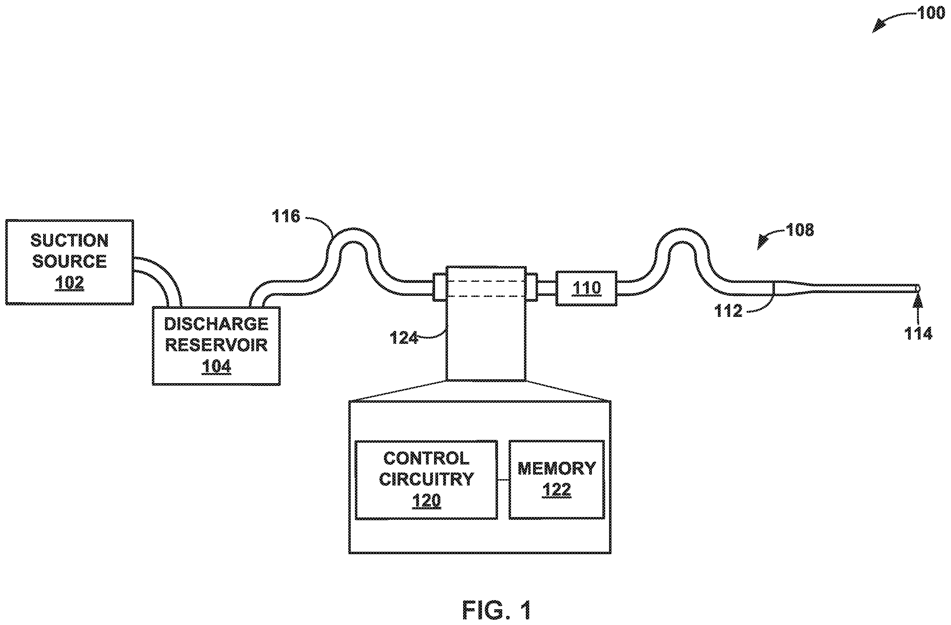

FIG. 1 is a schematic diagram illustrating an example medical aspiration system 100 including a suction source 102, a discharge reservoir 104, an aspiration catheter 108, and a flow switch 110. Aspiration system 100 may be used to treat a variety of conditions, including thrombosis. Thrombosis occurs when a thrombus (e.g., a blood clot or other material such as plaques or foreign bodies) forms and obstructs vasculature of a patient. For example, medical aspiration system 100 may be used to treat a pulmonary embolism or deep vein thrombosis, which may occur when a thrombus forms in a deep vein of a patient, such as in a leg of the patient.

Aspiration system 100 is configured to remove fluid via catheter 108, e.g., draw fluid from catheter 108 into discharge reservoir 104, via a suction force applied by suction source 102 to catheter 108 (e.g., to an inner lumen of catheter 108). Catheter 108 includes an elongated body 112 defining a catheter lumen (not shown in FIG. 1) and terminating in a distal opening 114. To treat a patient with thrombosis, a clinician may position distal opening 114 of catheter 108 in a blood vessel of the patient near the thrombus or other occlusion, and apply a suction force (also referred to herein as suction, vacuum force, negative pressure, or aspiration force) to the catheter 108 (e.g., to one or more lumens of the catheter) to engage the thrombus with suction force at distal opening 114 of catheter 108. For example, suction source 102 can be configured to create a negative pressure within the inner lumen of catheter 108 to draw a material from the inside the blood vessel into the catheter lumen via distal opening 114 of catheter 108. The negative pressure within the inner lumen can create a pressure differential between the inner lumen and the environment external to at least a distal portion of catheter 108 that causes the material, e.g., a thrombus, fluid (e.g., blood, saline introduced into the patient as part of the aspiration procedure, or the like), and/or other material, to be introduced from the blood vessel into the catheter lumen via catheter opening 114. For example, the fluid may flow from patient vasculature, into the catheter lumen via distal opening 114, and subsequently through aspiration tubing 116 (also referred to herein as "vacuum tube 116") into discharge reservoir 104.

Once distal opening 114 of aspiration catheter 108 has engaged a thrombus that is within a blood vessel, the clinician may remove aspiration catheter 108 with the thrombus held within opening 114 or attached to the distal tip of elongated body 112, or suction off pieces of the thrombus (or the thrombus as a whole) until the thrombus is removed from the blood vessel of the patient through a lumen of aspiration catheter 108 itself and/or through the lumen of an outer catheter in which aspiration catheter 108 is at least partially positioned. The outer catheter can be, for example, a guide catheter configured to provide additional structural support to the aspiration catheter. In some cases, aspiration of thrombus can be performed concurrently with use of a thrombectomy device, such as a thrombus removal basket, to facilitate removal of thrombus via mechanical thrombectomy as well as via aspiration.

As used herein, "suction force" is intended to include, within its scope, related concepts such as suction pressure, vacuum force, vacuum pressure, negative pressure, fluid flow rate, and the like. A suction force can be generated by a vacuum, e.g., by creating a partial vacuum within a sealed volume fluidically connected to catheter 108, or by direct displacement of liquid in catheter 108 and/or tubing 116 via (e.g.) a peristaltic pump, or otherwise. Accordingly, suction forces or suction as specified herein can be measured, estimated, computed, etc. without need for direct sensing or measurement of force. A "higher," "greater," or "larger" (or "lower," "lesser," or "smaller") suction force described herein may refer to the absolute value of the negative pressure generated by the suction source on a catheter or another component, such as a discharge reservoir 104.

In some examples, suction source 102 can comprise a pump (also referred to herein as "pump 102" or "vacuum source 102"). The suction source 102 can include one or more of a positive displacement pump (e.g., a peristaltic pump, a rotary pump, a reciprocating pump, or a linear pump), a direct-displacement pump (e.g., a peristaltic pump, or a lobe, vane, gear, or piston pump, or other suitable pumps of this type), a direct-acting pump (which acts directly on a liquid to be displaced or a tube containing the liquid), an indirect-acting pump (which acts indirectly on the liquid to be displaced), a centrifugal pump, and the like. An indirect-acting pump can comprise a vacuum pump, which displaces a compressible fluid (e.g., a gas such as air) from the evacuation volume (e.g., discharge reservoir 104, which can comprise a canister), generating suction force on the liquid. Accordingly, the evacuation volume (when present) can be considered part of the suction source. In some examples, suction source 102 includes a motor-driven pump, while in other examples, suction source 102 can include a syringe, and mechanical elements such as linear actuators, stepper motors, etc. As further examples, the suction source 102 could comprise a water aspiration venturi or ejector jet.

Aspiration system 100 includes control circuitry 120 configured to control a suction force applied by suction source 102 to catheter 108. For example, control circuitry 120 can be configured to directly control an operation of suction source 102 to vary the suction force applied by suction source 102 to the inner lumen of catheter 108, e.g. by controlling the motor speed, or stroke length, volume or frequency, or other operating parameters, of suction source 102. As another example, control circuitry 120 can be configured to control one or more functions of flow switch 110. Other techniques for modifying a suction force applied by suction source 102 to the inner lumen of catheter 108 can be used in other examples.

Control circuitry 120, as well as other processors, processing circuitry, controllers, control circuitry, and the like, described herein, may include any combination of integrated circuitry, discrete logic circuity, analog circuitry, such as one or more microprocessors, digital signal processors (DSPs), application specific integrated circuits (ASICs), or field-programmable gate arrays (FPGAs). In some examples, control circuitry 120 may include multiple components, such as any combination of one or more microprocessors, one or more DSPs, one or more ASICs, or one or more FPGAs, as well as other discrete or integrated logic circuitry, and/or analog circuitry. In some examples, control circuitry 120 may further include, additionally or alternatively to electric-based processors, one or more controls that operate using fluid motion power (e.g., hydraulic power) in combination with or in addition to electricity. For example, control circuitry 120 can include a fluid circuit comprising a fluid circuit comprising a plurality of fluid passages and switches arranged and configured such that, when a fluid (e.g., liquid or gas) flows through the passages and interacts with the switches, the fluid circuit performs the functionality of control circuitry 120 described herein.

Memory 122 may store program instructions, such as software, which may include one or more program modules, which are executable by control circuitry 120. When executed by control circuitry 120, such program instructions may cause control circuitry 120 to provide the functionality ascribed to control circuitry 120 herein. The program instructions may be embodied in software and/or firmware. Memory 122, as well as other memories described herein, may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media. Although control circuitry 120 and memory 122 are shown in FIG. 1 as being in a common housing, in other examples, control circuitry 120 and/or memory 122 can be physically separate from each other.

With some other aspiration systems, some amount of a body fluid may be incidentally withdrawn during the aspiration procedure. For instance, while approaching and aspirating a thrombus with a distal opening of the catheter, the clinician may incidentally aspirate and remove a volume of the patient's blood, e.g., that is not inherently necessary to withdraw as part of the procedure. As described herein, aspiration system 100 includes flow switch 110 (also referred to herein as "valve 110"), configured to mitigate or prevent the incidental withdrawal of the patient's blood during aspiration.

More specifically, flow switch 110 is configured to passively close to restrict aspiration of a body fluid from a body of a patient. Flow switch 110 is configured to be fluidically coupled to aspiration catheter 108 at a distal portion of flow switch 110, and fluidically coupled to vacuum tubing 116 at a proximal portion of flow switch 110, thereby defining a continuous fluid flow pathway (e.g., a continuous lumen) through aspiration system 100. In general, flow switch 110 is configured to passively close the continuous pathway to inhibit or disrupt a proximal flow of a body fluid, such as blood. Once the proximal fluid flow is reduced below a threshold flow rate, or is no longer present, within flow switch 110 (e.g., is not flowing proximally through the inner lumen of elongated body 112 of catheter 108), flow switch 110 is configured to passively re-open the continuous pathway, thereby enabling effective aspiration of thrombus material into distal catheter opening 114 and proximally through a catheter lumen defined by elongated body 112 of catheter 108.

In some examples, flow switch 110 includes a housing defining an internal cavity and a plug disposed within the internal cavity. In some examples, the plug is positioned between opposing proximal and distal springs (not shown in FIG. 1). In some such examples, each spring is operatively coupled to an opposing side of the plug. In other such examples, the plug is operatively coupled to only one of the springs, but configured to contact the other spring in certain configurations. In other examples, flow switch 110 includes either a proximal spring or a distal spring, but not both.

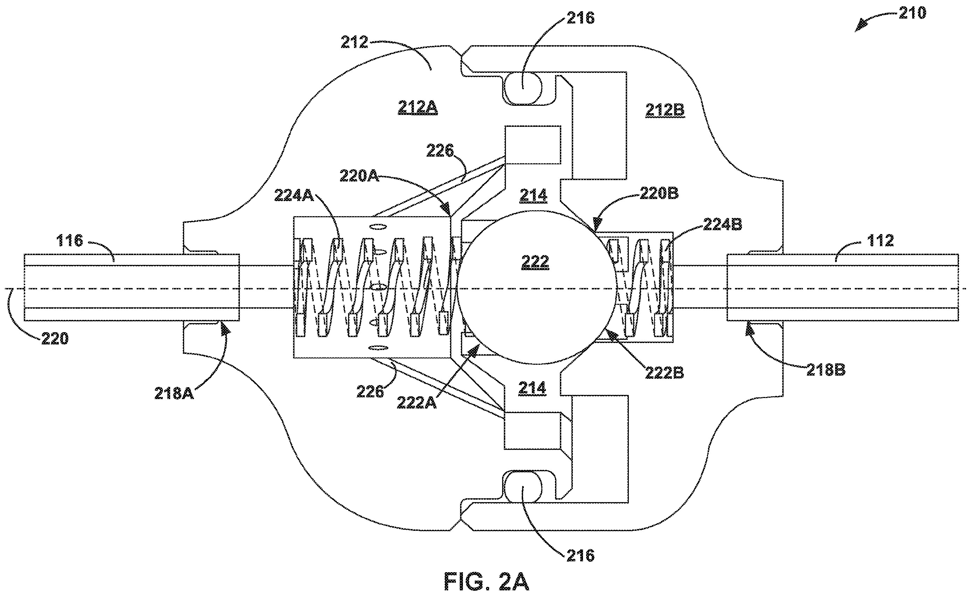

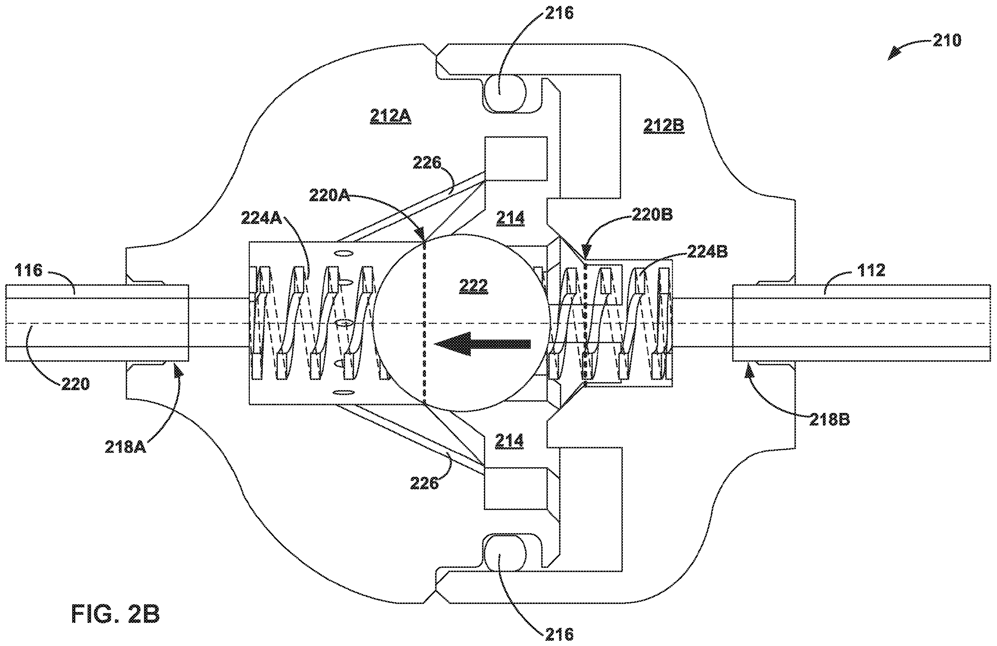

In the presence of a proximally oriented, above-threshold fluid flow through the internal cavity of flow switch 110, the fluid imparts a corresponding drag force onto a distal-facing surface of the plug, causing the plug to move proximally to seal a proximal opening of the housing of flow switch 110, thereby restricting further fluid flow in a proximal direction through the housing. For example, FIGS. 2A and 2B depict cross-sectional side views of an example flow switch 210, which is an example of flow switch 110 of FIG. 1. In the example of FIG. 2A, flow switch 210 is depicted in an "open" configuration, in which an aspirated substance is freely able to flow proximally (e.g., right-to-left, from the perspective of FIG. 2A) through switch 210 along a continuous pathway generally indicated by central axis 220. In the example of FIG. 2B, flow switch 210 is depicted in a "closed" configuration, in which the continuous pathway is obstructed to block fluid flow through flow switch 210.

As shown in FIGS. 2A and 2B, flow switch 210 includes a housing 212 that defines an internal cavity 214. In some examples, such as the example depicted in FIG. 2A, but not all examples, housing 212 includes both a proximal housing portion 212A and a distal housing portion 212B. Proximal and distal housing portions 212A, 212B may be shaped and sized so as to be mutually interlocking or otherwise mechanically connected, and fluidically sealed by an O-ring 216 so as to define the internal cavity 214.

As defined by housing 212, internal cavity 214 extends distally (e.g., left-to-right, from the perspective of FIG. 2A) from a proximal housing opening 218A to a distal housing opening 218B along a longitudinal axis 220. Housing 212 is configured to removably interconnect with vacuum tubing 116 at proximal housing opening 218A, and to removably interconnect with elongated body 112 of catheter 108 (FIG. 1) at distal housing opening 218B. Thus, when flow switch 210 is in an "open" configuration, internal cavity 214 fluidically connects vacuum tubing 116 and elongated body 112 of catheter 108. That is, a fluid pathway is defined by vacuum tubing 116, elongated body 112, and flow switch.

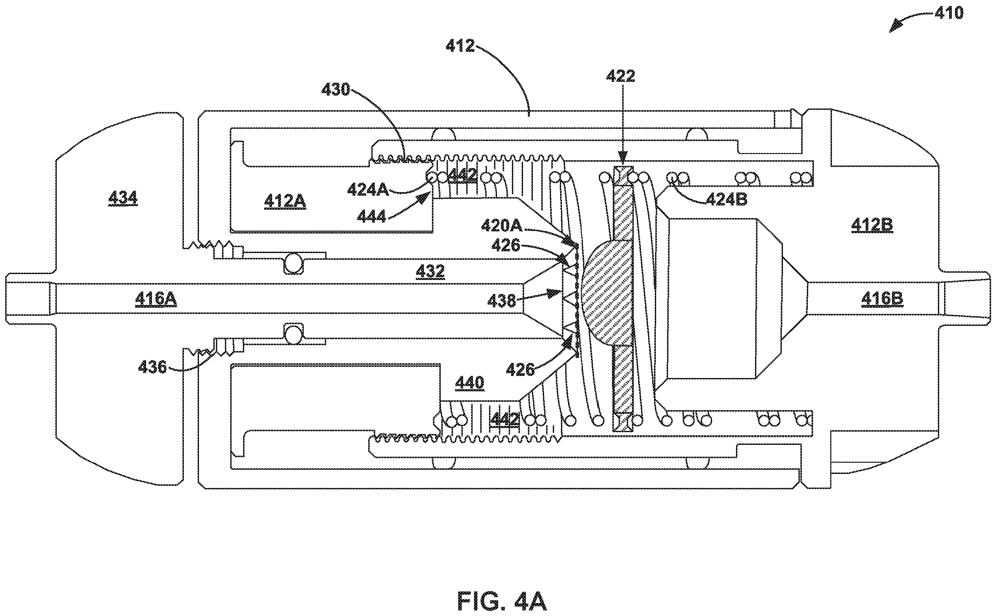

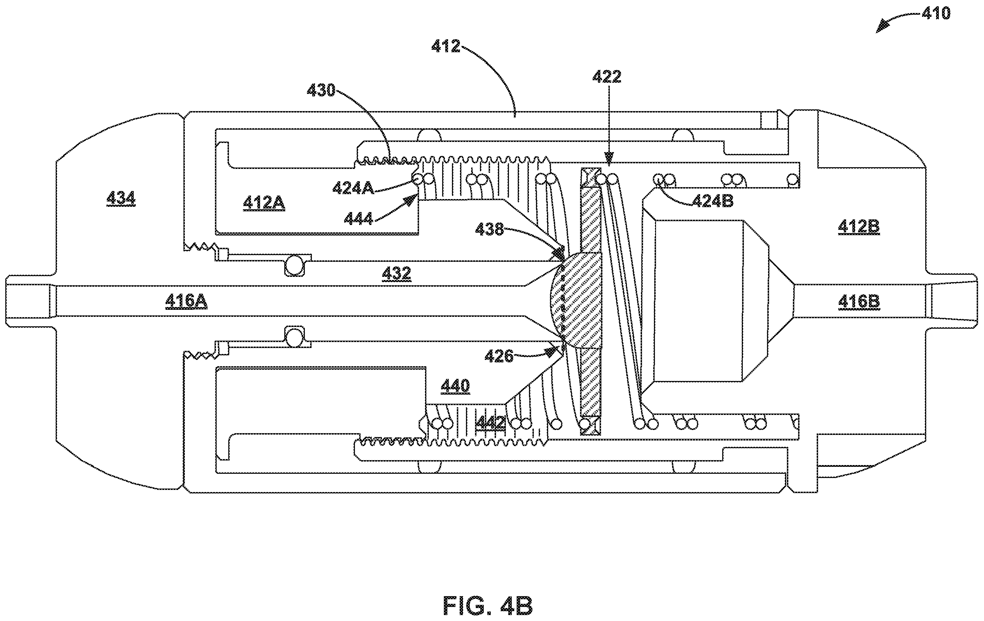

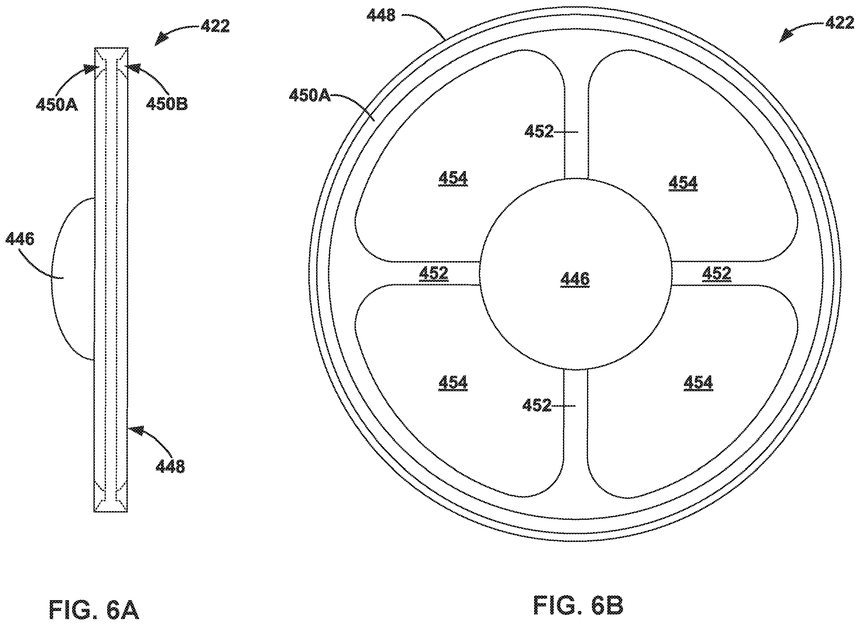

Housing 212 of flow switch 210 defines a proximal valve opening 220A and a distal valve opening 220B, the openings 220A, 220B defining openings into cavity 214. As described herein, flow switch 210 further includes a plug 222 positioned within internal cavity 214. In the example shown in FIGS. 2A-3, plug 222 includes a substantially spherical object, however, as detailed further below with respect to FIGS. 4, 6A, and 6B, plug 222 can have other suitable configurations, such as a hemispherical or conical shape.

Plug 222 is movably positioned or suspended within internal cavity 214 by a biasing mechanism. As used herein, a "biasing mechanism" includes any suitable device configured to retain plug 222 in an equilibrium position in the absence of outside forces acting upon the plug, or equivalently, to passively (e.g., without human intervention) restore plug 222 toward the equilibrium position when outside forces acting upon plug 222 are removed. In the example shown in FIGS. 2A and 2B, the biasing mechanism includes a proximal spring 224A and a distal spring 224B (collectively, "springs 224") operatively coupled (e.g., configured to apply a biasing force) to an opposing side of plug 222 from proximal spring 224A. Additionally or alternatively, the biasing mechanism may include, as non-limiting examples, one or more elastic bands operatively coupled to plug 222, one or more magnetic objects configured to attract or repel plug 222 toward the equilibrium position, or the like.

In some examples, the biasing mechanism may include both a proximal spring 224A and a distal spring 224B, but plug 222 may be operatively coupled to only the proximal spring 224A or the distal spring 224B, but not both. For instance, plug 222 may be operatively coupled to distal spring 224B but not to proximal spring 224A. In some such examples, plug 222 may be configured to contact proximal spring 224A in certain configurations of flow switch 210, such as when flow switch 210 is in the "closed" configuration and plug 222 is biased proximally within internal cavity 214. Alternatively, plug 222 may be operatively coupled to proximal spring 224A but not to distal spring 224B, wherein plug 222 is configured to contact distal spring 224B while flow switch 210 is in the "open" configuration and plug 222 is biased distally.

In other examples, the biasing mechanism of flow switch 210 may include either a proximal spring 224A or a distal spring 224B, but not both. For instance, flow switch 210 may include only proximal spring 224A and not distal spring 224B, wherein a proximal fluid flow through internal cavity 214 applies a drag force onto plug 222 to compress proximal spring 224A and bias plug 222 proximally. As another example, flow switch 210 may include only distal spring 224B and not proximal spring 224A, wherein a proximally oriented fluid flow through internal cavity 214 applies a drag force onto plug 222 to expand distal spring 224B and bias plug 222 proximally. One or both of springs 224 can include any suitable type of spring, such as, but not limited to, a coil spring, a wave spring, a leaf spring, or the like. Further, as discussed in further detail below, springs 224 can be tension springs or compression springs.

In some examples in which the biasing mechanism includes both proximal spring 224A and distal spring 224B, and in which both springs 224 are operatively coupled to a respective opposing side of plug 222, the proximal and distal springs 224A, 224B may both include or be "tension" springs. For instance, while plug 222 is suspended in the equilibrium position between proximal and distal springs 224, both of the springs 224 may be in a respective at-least-partially expanded configuration, thereby applying a respective tension force onto either side of plug 222. That is, when a proximal fluid flow through internal cavity 214 applies an above-threshold drag force onto plug 222, the resulting proximal motion of plug 222 will further expand distal spring 224B and will enable proximal spring 224A to compress toward its spring-equilibrium position.

In other examples, such as the example depicted in FIGS. 2A and 2B, the proximal and distal springs 224 may both include or be "compression" springs. For instance, while plug 222 is suspended in the equilibrium position between proximal and distal springs 224, both of the springs 224 may be in a respective at-least-partially compressed configuration, thereby applying a respective compression force on either side of plug 222. That is, when a proximal fluid flow through internal cavity 214 applies an above-threshold drag force onto plug 222, the resulting proximal motion of plug 222 will further compress proximal spring 224A and will enable distal spring 224B to expand toward its spring-equilibrium position. For ease of description, one of tension or compressive springs may be referred to in the description herein, but in other examples, springs 224 can be configured to apply the other of compressive or tension forces to plug 222.

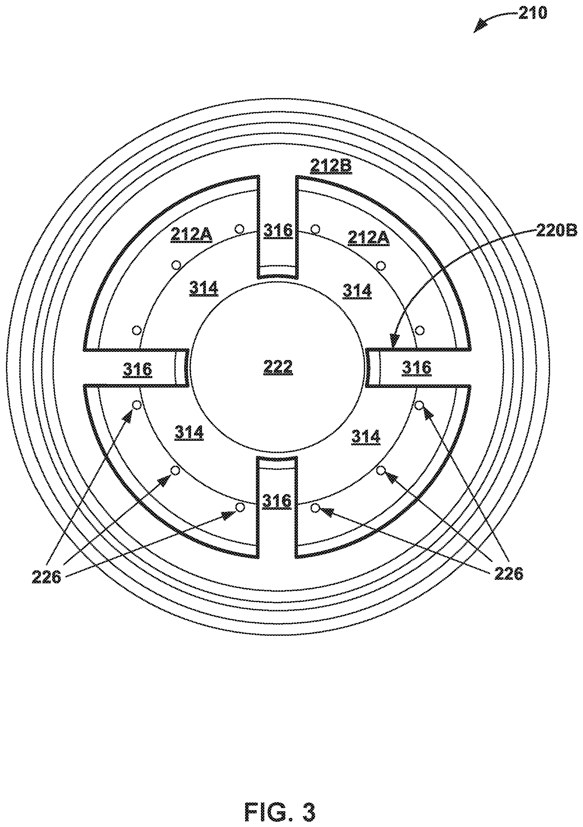

In this way, in the "open" configuration of flow switch 210 depicted in FIG. 2A, springs 224 each impart a respective compression force (or alternatively, a respective tension force, as described above) onto plug 222 that, in the absence of additional external forces, bias plug 222 distally toward distal valve opening 220B. Springs 224 can directly (e.g., direct physical contact) or indirectly contact plug 222 (e.g., via an intermediary structure that transmits the force from the respective spring 224 to plug 222). Unlike proximal valve opening 220A, which defines a generally circular opening that substantially conforms to a cross-sectional profile of plug 222, as depicted in the cross-sectional transverse view of FIG. 3, distal valve opening 220B defines a substantially irregular cross-sectional shape that does not substantially conform to a cross-sectional profile of plug 222. In other words, when plug 222 is distally biased toward distal valve opening 220B, flow switch 210 includes one or more gaps 314 (FIG. 3) enabling a fluid or other material to flow proximally around plug 222 and through internal cavity 214.

As described above, during use, a clinician may position distal opening 114 of catheter 108 within vasculature or another hollow anatomical structure of a patient, near a target treatment site (e.g., a part of a blood vessel including a thrombus). The clinician may activate suction source 102 to aspirate occlusive material into distal opening 114 of catheter 108. In some instances, distal opening 114 may be only partially occluded via contact with thrombus material, or in other instances, may not be occluded by thrombus material at all, such as when distal opening 114 is not yet positioned into engagement with the thrombus within the patient. In such instances, suction source 102 may begin to aspirate an amount of a body fluid, such as blood, into distal opening 114, through elongated body 112 and aspiration tubing 116, and into discharge reservoir 104 to be subsequently discarded. It may be undesirable to remove relatively large volumes of body fluid from the patient during the aspiration procedure. Flow switch 110 is configured to passively transition from the "open" configuration depicted in FIG. 2A to the "closed" configuration depicted in FIG. 2B to restrict or prevent further aspiration of the body fluid from the body of the patient.

For instance, while the body fluid is aspirated proximally through cavity 214, at least some of the fluid will contact a distal-facing surface 222B of plug 222, thereby imparting a proximal drag force onto plug 222. As indicated in FIG. 2B, when the proximal drag force is sufficient to overcome the threshold compression forces imparted by proximal and distal springs 224A, 224B, plug 222 will translate proximally toward proximal valve opening 220A. In this way, proximal spring 224A will compress from a less-compressed configuration to a more-compressed configuration, thereby imparting a greater distal compression force onto a proximal side 222A of plug 222. Similarly, distal spring 224B will expand from a more-compressed configuration toward a less-compressed configuration, thereby imparting a lesser proximal compression force onto distal side 222B of plug 222. Depending upon both the length and the spring constant of distal spring 224B, in some examples, distal spring 224B may continue to expand past its own spring-equilibrium position into an expanded configuration, thereby imparting a distal tension force onto distal side 222B of plug 222.

As described above, proximal valve opening 220A is shaped so as to substantially conform (e.g., conform or nearly conform to the extent permitted by manufacturing tolerances) to a cross-sectional profile of plug 222 such that, when plug 222 is biased proximally against proximal valve opening 220A, proximal valve opening 220A is substantially occluded by plug 222 (e.g., fully occluded or nearly fully occlude) and, by extension, the continuous pathway or lumen through internal cavity 214 is likewise occluded, preventing further fluid flow through flow switch 210. This may help reduce or even stop the further removal of the body fluid from the body of the patient.

Once plug 222 is initially biased against proximal valve opening 220A by the fluid draft force, a pressure seal or vacuum seal may form to retain plug 222 in place in the proximal "closed" position. For instance, the aspiration force from suction source 102 can provide a relatively low-pressure region on the proximal side of plug 222, and the venous blood pressure of the patient can provide a relatively high-pressure region on the distal side of plug 222, thereby proximally biasing plug 222 and retaining flow switch 210 in the "closed" configuration shown in FIG. 2B.

As shown in FIGS. 2A, 2B, and 3, housing 212 defines a plurality of leak channels 226, distributed circumferentially around proximal valve opening 220A, and extending between a relatively central region of internal cavity 214 and a more-proximal region of internal cavity 214. Leak channels 226 enable a relatively small amount of fluid to bypass or circumvent proximal valve opening 220A, even when proximal valve opening 220A is occluded by plug 222 (e.g., when flow switch 210 is in a closed configuration). Although the amount of fluid aspirated through leak channels 226 may be relatively negligible, the associated transmission of fluid pressure through leak channels 226 enables flow switch 210 to revert back to the open configuration depicted in FIG. 2A once the fluid flow through the channels 226 is removed.

For instance, once the clinician contacts a more-solid thrombus material with distal opening 114 of catheter 108, the fluid flow into elongated body 112 may be disrupted or entirely prevented (e.g., when distal catheter opening 114 is pressure-sealed against the occlusive material by suction source 102). In such instances, the fluid pressure on the distal side of plug 222 may begin to drop, and eventually equalize with the fluid pressure on the proximal side of plug 222 via leak channels 226. At a threshold pressure differential, the tension force and/or compression force from springs 224 will be sufficient to break the seal holding plug 222 against proximal valve opening 220A and springs 224 will distally translate plug 222 back toward distal valve opening 220B and the open configuration of flow switch 210.

In this way, the devices, systems, and techniques of this disclosure enable a clinician to use aspiration catheter 108 to "search" or "probe" for occlusive material within the vasculature of a patient (e.g., with the assistance of visual fluoroscopy) without withdrawing an excessive amount of the patient's blood or other body fluid in the process. In other words, flow switch 210 (and the other example flow switches described herein) enables a clinician to primarily aspirate more-solid or more-viscous thrombus material, and not more-liquid or less-viscous patient material, even absent a precise alignment and occlusion of distal catheter opening 114 with the thrombus.

FIG. 3 is a cross-sectional view of the example flow switch of FIGS. 2A and 2B, wherein the cross-section is taken orthogonal to longitudinal axis 220. As described above, distal valve opening 220B (indicated by the thick black line in FIG. 3) is a geometrically irregular shape that does not conform to a cross-sectional profile of plug 222 when plug 222 is biased distally against distal valve opening 220B. More specifically, in the example of FIG. 3, distal housing portion 212B defines a plurality of plug supports 316 extending radially inward into internal cavity 214 (FIGS. 2A and 2B). Plug supports 316 are configured to position plug 222 approximately radially centered within internal cavity 214, while enabling plug 222 to move axially (e.g., proximally and distally along longitudinal axis 220) in response to the presence or absence of a fluid flow. As shown in FIG. 3, each circumferentially adjacent pair of plug supports 316 defines a respective gap 314 therebetween. In some examples, but not all examples, supports 316 may be sized and shaped such that each gap 314 has a cross-sectional area that is larger than a cross-sectional area of the inner lumen of the elongated body 112 of catheter 108. In such configurations, any thrombus material aspirated into internal cavity 214 (e.g., through gaps 314) may be substantially unlikely to occlude or jam internal cavity 214.

Although four plug supports 316 that are equally spaced around plug 222 are shown in FIG. 3, in other examples, flow switch 210 can include any suitable number of plug supports, such as two, three, or more than four, or one having a shape that is configured to simultaneously engage plug 222 and define gaps 314. The plug supports can be evenly distributed or unevenly distributed about a common axis (e.g., longitudinal axis 220).

FIGS. 4A and 4B are cross-sectional side views of another flow switch 410, which is another example of flow switch 110 of FIG. 1. Flow switch 410 may also be an example of flow switch 210 of FIGS. 2A-3, except for the differences noted herein. For instance, similar to flow switch 210, flow switch 410 includes a housing 412 having proximal and distal housing portions 412A, 412B, respectively, a plug 422 suspended between proximal and/or distal springs 424A, 424B (or other suitable biasing mechanism), and leak channels 426. FIG. 4A depicts flow switch 410 in an "open" configuration, and FIG. 4B depicts flow switch 410 in a "closed" configuration. Additionally, as detailed further below, FIG. 4A depicts a configuration of flow switch 410 with fully "open" leak channels 426, and FIG. 4B depicts a configuration of flow switch 410 with fully "closed" leak channels 426.

As shown in FIGS. 4A and 4B, proximal and distal housing portions 412A, 412B are radially nested (e.g., include portions that overlap along an axial direction) and threadably coupled via first threading 430 (or "threading 430"). Threading 430 enables a user (e.g., the clinician) to axially translate proximal and distal housing portions 412A, 412B relative to one another, thereby axially collapsing or expanding, respectively, the internal cavity 414 therebetween, and by extension, axially compressing or expanding the springs 424A, 424B extending between proximal-facing and distal-facing surfaces of internal cavity 414. In this way, threading 430 enables the user to control a "closing sensitivity" of flow switch 410. In other words, by threadably rotating housing portions 412A, 412B relative to one another, the user can manipulate the base or default tension force or compression force applied to plug 422 by springs 424A, 424B. For instance, when plug 422 is suspended under an increased tension force or compression force from springs 424, flow switch 410 requires an increased threshold minimum drag force (e.g., an increased threshold flow rate) from a fluid flow through internal cavity 414 in order to overcome the tension force or compression force and move plug 422 proximally to close switch 410 (i.e., fully or partially block fluid flow through switch 410). Conversely, when plug 422 is suspended under a decreased tension force or compression force, respectively, from springs 424, flow switch 410 requires a decreased minimum threshold drag force (e.g., a decreased threshold flow rate) from a fluid flow through internal cavity 414 to overcome the tension force or compression force and move plug 422 proximally to close the switch.

In some examples, flow switch 410 includes a mechanism that enables a user to adjust or modify fluid flow through leak channels 426 in order to control a "responsiveness" of flow switch 410, or in other words, a duration or delay between the time at which a fluid flow is either introduced or removed from internal cavity 414, and the time at which the switch 410 re-opens in response to the absence of the fluid flow. In some examples, flow switch 410 may include a selector switch (e.g., a rotatable switch) that closes or opens different leak channels 426. For instance, in examples in which all of leak channels 426 have the same inner diameter (or other measurement of cross-sectional area), the selector switch may increase the responsiveness of flow switch 410 by opening additional leak channels 426, or may decrease the responsiveness of flow switch 410 by closing leak channels 426. In other examples in which different leak channels 426 have different cross-sectional areas, the selector switch may increase the responsiveness of flow switch 410 by opening larger leak channels and closing smaller channels, or may decrease the responsiveness of flow switch 410 by closing larger leak channels and opening smaller leak channels.

In the example depicted in FIGS. 4A and 4B, the selector switch includes an adjustable needle valve 432 with an external adjustment screw 434. Needle valve 432 is configured to enable the user to control the responsiveness of flow switch 410 by fluidically coupling (or "opening") leak channels 426 to proximal lumen 416A (as shown in FIG. 4A) or by fluidically occluding (or "closing") leak channels 426 from proximal lumen 416B (as shown in FIG. 4B).

More specifically, needle valve 432 is threadably coupled to proximal housing portion 412A via second threading 436. In the "proximal" or "open" configuration of needle valve 432 shown in FIG. 4A, the distal-most end 438 of needle valve 432 is located proximally from the proximal valve opening 420A, such that leak channels 426 are fluidically coupled to proximal lumen 416A. In other words, if flow switch 410 were in a closed configuration in which plug 422 were proximally sealed against proximal valve opening 420A (indicated by the thick black dashed line in FIG. 4A), a small amount of fluid flow could bypass plug 422 through leak channels 426. Conversely, in the "distal" or "closed" configuration of needle valve 432 shown in FIG. 4B, the distal-most end 438 of needle valve 432 is either axially aligned with proximal valve opening 420A, or in some examples, positioned distally to the proximal valve opening 420A, such that plug 422 directly contacts and seals the distal-most end 438 of needle valve 432, thereby fluidically occluding leak channels 426 and proximal lumen 416A. By manipulating adjustment screw 434, the user can select the responsiveness of the leak channels 426 from anywhere between the open configuration (FIG. 4A) and the closed configuration (FIG. 4B).

In some examples (not shown in FIGS. 4A and 4B), flow switch 410 may additionally or alternatively include a complete bypass mechanism that enables the user to hold flow switch 410 in the open configuration (FIG. 4A) regardless of the presence of a fluid flow through internal cavity 414. In some examples, as shown in FIGS. 4A and 4B, each of proximal and distal springs 424A, 424B may include a nested pair of springs. In other examples, each of proximal and distal springs 424A, 424B may include more than two springs helically nested together.

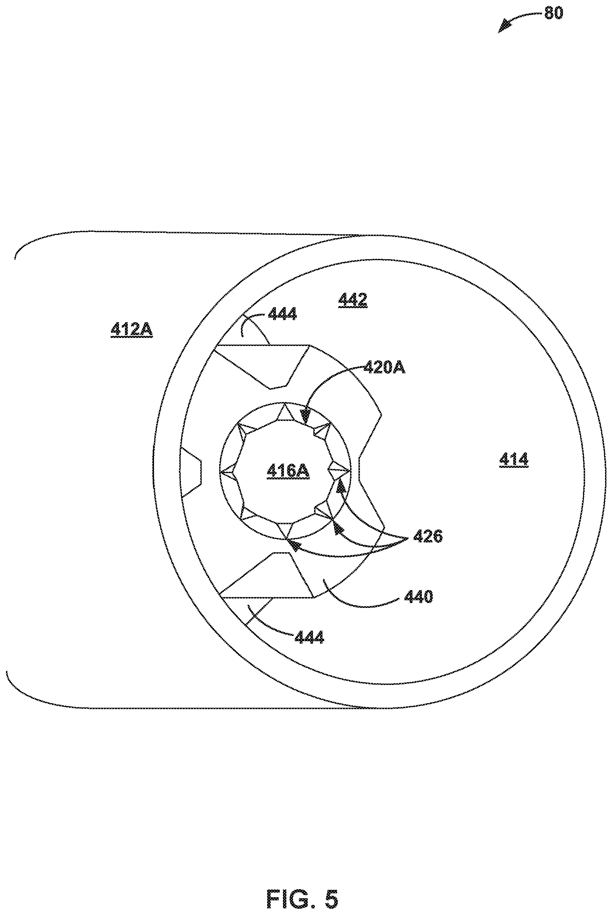

FIG. 5 is a perspective view of the proximal housing portion 412A of the passive flow switch 410 of FIGS. 4A and 4B. As shown in FIG. 5, proximal housing portion 412 defines a protrusion 440 that in turn defines proximal valve opening 420A to proximal lumen 416A. Protrusion 440 is approximately radially centered within internal cavity 414, thereby defining an annular portion 442 of cavity 414, located circumferentially around protrusion 440. The annular portion 442 of cavity 414 is configured to receive a proximal portion of proximal spring 424A that contacts proximal cavity wall 444. In such configurations, proximal spring 424A is not located within the primary fluid pathway into proximal valve opening 420A to proximal lumen 416A, providing for improved aspiration thrombus material and reduced probability for blockage of the pathway.

FIGS. 6A and 6B are side and end views, respectively, of an example of plug 422 of flow switch 410 of FIGS. 4A and 4B. Unlike plug 222 of FIGS. 2A-3, which includes a substantially spherical shape configured to both fluidically seal proximal valve opening 220A as well as couple to proximal and distal springs 224A, 424B, as shown in FIGS. 6A and 6B, plug 422 of includes physically distinct components configured to perform the equivalent functions, respectively. More specifically, plug 422 includes a generally hemispherical portion 446 configured to seal proximal valve opening 420A, and an annular portion 448 configured to receive and/or couple to proximal and distal springs 424A, 424B.

In some examples, annular portion 448 of plug 422 defines a proximal annular groove 450A configured to receive a distal end of proximal spring 424A. Similarly, annular portion of plug 422 may define a distal annular groove 450B configured to receive a proximal end of distal spring 424B. Hemispherical portion 446 and annular portion 448 may be operatively coupled by a plurality of radial struts 452 defining gaps 454 therebetween for fluid and/or thrombus material to travel through while flow switch 410 is in the open configuration shown in FIG. 4A.

In some examples, radial struts 452 may be formed from a substantially rigid material, such that hemispherical portion 446, annular portion 448, and radial struts 452 collectively define a structurally coherent unit. In other examples, radial struts 452 may be formed from a substantially elastic material, such that radial struts 452 define a biasing mechanism for plug 422, as described above with respect to plug 222. For instance, radial struts 452 may include elastic bands that, in the absence of outside forces applied to hemispherical portion 446, suspend hemispherical portion 446 in a (distal) equilibrium position, defining the "open" configuration of flow switch 410. In the presence of a fluid flow through internal cavity 414, a resulting above-threshold drag force applied to the distal surface of hemispherical portion 446 can overcome the respective compression and/or tension forces from radial struts 452, causing hemispherical portion 446 to move proximally. In such examples, annular portion 448 may be either fixed in place relative to internal cavity 414, or in other examples, may be entirely absent (e.g., radial struts 452 may extend between an interior surface of housing 412 and hemispherical portion 446. Additionally or alternatively, in such configurations, flow switch 410 may not include either or both of proximal and distal springs 424A, 424B, and radial struts 452 may provide the plug-biasing functionality alone.

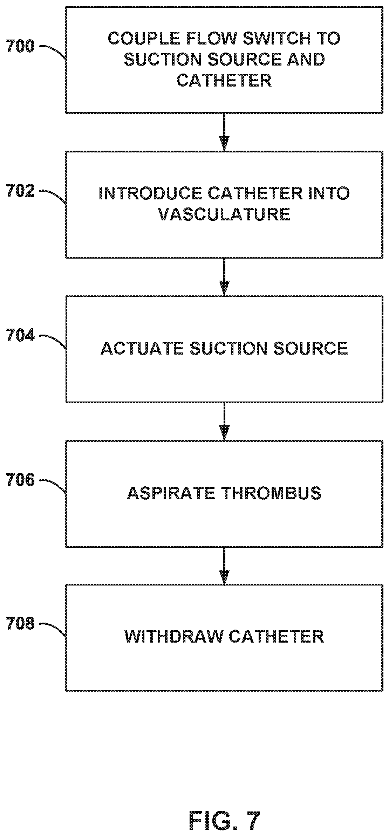

Passive flow switches as described herein may be formed using any suitable technique and can be used in any suitable medical procedure. FIG. 7 is a flow diagram of an example technique for using the aspiration systems and flow switches described herein. The technique of FIG. 7 is described with reference to the various aspects of aspiration system 100 of FIG. 1 and flow switch 210 of FIGS. 2A-3 for illustrative purposes, however, such descriptions are not intended to be limiting. The technique of FIG. 7 may be used with other aspiration systems and/or flow switches described herein, or aspiration system 100 and/or flow switch 210 of FIG. 1 may be used using techniques other than those described with reference to FIG. 7.

In accordance with the technique shown in FIG. 7, a clinician fluidically couples flow switch 210 to a suction source 102 and to the inner lumen of a catheter 108 (700). For example, the clinician may mechanically and fluidically connect vacuum tube 116 to a proximal opening 218A of flow switch 210. The clinician may also mechanically and fluidically connect elongated body 112 of catheter 108 to distal opening 218B of flow switch 210, so as to define a continuous fluid flow pathway through the inner lumen of catheter 108, the internal cavity 214 of flow switch 210, and the inner lumen of vacuum tube 116.

Prior to or after coupling flow switch 210 to catheter 108 and suction source 102, the clinician introduces catheter 108 into vasculature of a patient (702) and navigates catheter 108 to a target treatment site within a patient. In some examples, the clinician navigates catheter 108 to the target site with the aid of a guidewire, guide catheter or another guide member.

After distal opening 114 of catheter 108 is positioned as desired proximate a thrombus in the vasculature (e.g., as indicated via fluoroscopic imagery), control circuitry 120, alone or based on input from a user received via a user input device, controls suction source 102 to generate a suction force within the inner lumen of catheter 108 (704).

In some instances, distal opening 114 of catheter 108 may initially be positioned near thrombus material, but may not be positioned against (e.g., occluded by) the thrombus material. In such instances, the suction force from suction source 102 may begin to aspirate a patient fluid, such as blood, into catheter 108, generating a proximal fluid flow through internal cavity 214 of flow switch 210. In such cases, the fluid flow may impart a drag force onto a distal-facing surface 222B of plug 222 within internal cavity 214. When this drag force is sufficiently strong to overcome a threshold compression force (or a threshold tension force in other examples) from springs 224A, 224B, plug 222 may translate proximally and seal proximal valve opening 220A, thereby restricting or preventing further fluid flow through flow switch 210.

The clinician may continue to manipulate distal opening 114 of catheter 108 until distal opening 114 is positioned against, and occluded by, thrombus material. At such time, the fluid flow into catheter 108 will be disrupted by the thrombus material at distal opening 114, and the fluid pressure on a distal side of plug 222 may begin to drop. Further, the fluid pressure on both the proximal and distal sides of plug 222 may begin to equalize via leak channels 226. When the fluid pressure is sufficiently balanced, the compression force (or tension forces in other examples) from springs 224 will distally bias plug 222 back toward the distal valve opening 220B, such that flow switch 210 reverts to an open configuration. With flow switch 210 open, the technique of FIG. 7 further the clinician may proceed to aspirate the thrombus (706) and remove catheter 108 from the vasculature of the patient once the procedure is complete (708).

The techniques described in this disclosure, including those attributed to control circuitry 120, or various constituent components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, embodied in programmers, such as clinician or patient programmers, medical devices, or other devices. Processing circuitry, control circuitry, and sensing circuitry, as well as other processors and controllers described herein, may be implemented at least in part as, or include, one or more executable applications, application modules, libraries, classes, methods, objects, routines, subroutines, firmware, and/or embedded code, for example. In addition, analog circuits, components and circuit elements may be employed to construct one, some or all of the control circuitry 120, instead of or in addition to the partially or wholly digital hardware and/or software described herein. Accordingly, analog or digital hardware may be employed, or a combination of the two. Whether implemented in digital or analog form, or in a combination of the two, control circuitry 120 can comprise a timing circuit.

In one or more examples, the functions described in this disclosure may be implemented in hardware, software, firmware, or any combination thereof. If implemented in software, the functions may be stored on, as one or more instructions or code, a computer-readable medium and executed by a hardware-based processing unit. The computer-readable medium may be an article of manufacture including a non-transitory computer-readable storage medium encoded with instructions. Instructions embedded or encoded in an article of manufacture including a non-transitory computer-readable storage medium encoded, may cause one or more programmable processors, or other processors, to implement one or more of the techniques described herein, such as when instructions included or encoded in the non-transitory computer-readable storage medium are executed by the one or more processors. Example non-transitory computer-readable storage media may include RAM, ROM, programmable ROM (PROM), erasable programmable ROM (EPROM), electronically erasable programmable ROM (EEPROM), flash memory, a hard disk, a compact disc ROM (CD-ROM), a floppy disk, a cassette, magnetic media, optical media, or any other computer readable storage devices or tangible computer readable media.

In some examples, a computer-readable storage medium comprises non-transitory medium. The term "non-transitory" may indicate that the storage medium is not embodied in a carrier wave or a propagated signal. In certain examples, a non-transitory storage medium may store data that can, over time, change (*e.g.,* in RAM or cache).

The functionality described herein may be provided within dedicated hardware and/or software modules. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components. Also, the techniques could be fully implemented in one or more circuits or logic elements.

Various aspects of devices, systems, and methods have been described. The invention is defined by the following claims.

## Claims

1. A flow switch comprising:
a housing (212, 412) defining an internal cavity (214, 414) and proximal and distal openings (220A, 220B) to the internal cavity (214, 414), wherein the internal cavity (214, 414) is configured to receive an aspirated fluid flow from a catheter; and
a plug (222, 422) disposed within the internal cavity (214, 414)
wherein the plug (222,422) is configured to:
move proximally, in response to an above-threshold drag force from the fluid flow applied to a distal-facing surface (222B) of the plug (222, 422), to close the proximal opening (220A, 420A); and
move distally to open the proximal opening (220A, 420A) in response to an absence of the fluid flow within the internal cavity (214, 414).

2. The flow switch of claim 1, further comprising:
a distal spring (224B, 424B) positioned between the distal-facing surface of the plug and an interior surface of the housing (212, 412); and
a proximal spring (224A, 424A) positioned between a proximal-facing surface of the plug (222, 422) and the interior surface of the housing (212, 412), wherein the proximal and distal springs are configured to bias the plug (222, 422) distally away from the proximal opening (220A, 420A) in the absence of the aspirated fluid flow within the internal cavity.

3. The flow switch of claim 2, further comprising an input mechanism configured to modify a compression or expansion force applied by the proximal spring or the distal spring to the plug (222, 422) to modify a magnitude of the drag force required move the plug (222, 422) proximally to close the proximal opening (220A, 420A).

4. The flow switch of claim 3, wherein the input mechanism comprises a threaded rotatable element.

5. The flow switch of any of the claims 2 to 4, wherein the internal cavity defines a fluid flow path and an annular space oriented radially outward from the fluid flow path, wherein the proximal and distal springs are disposed within the annular space.

6. The flow switch of any of the claims 1 to 5, wherein the housing (212, 412) further defines a plurality of leak channels extending from a distal cavity portion of the internal cavity distal to the plug (222, 422), to a proximal cavity portion of the internal cavity proximal to the rounded plug (222, 422), wherein the plurality of leak channels are configured to modulate a fluid-pressure differential between the proximal cavity portion and the distal cavity portion.

7. The flow switch of claim 6, further comprising a selector switch configured to open or close different leak channels of the plurality of leak channels to modulate a time delay between a removal of the fluid flow within the internal cavity and a re-opening of the proximal opening (220A, 420A) by the plug (222, 422).

8. The flow switch of any of the claims 6 or 7, further comprising an input mechanism configured to open or occlude at least one leak channel of the plurality of leak channels to modulate a time delay between a removal of the fluid flow within the internal cavity and a re-opening of the proximal opening (220A, 420A) by the plug (222, 422).

9. The flow switch of claim 8, wherein the input mechanism comprises an adjustable needle valve and an external adjustment screw.

10. The flow switch of any of the claims 6 to 9, wherein the fluid-pressure differential is configured to retain the flow switch in a closed configuration by at least holding the plug (222, 422) proximally over the proximal opening (220A, 420A).

11. The flow switch of any of the claims 6 to 10, wherein the leak channels of the plurality of leak channels are distributed circumferentially around a central longitudinal axis of the flow switch.

12. The flow switch of any of the claims 1 to 11, wherein the housing (212, 412) is configured to fluidically couple to proximal aspiration tubing at the proximal opening (220A, 420A), and to fluidically couple to an elongated body of the catheter at the distal opening.

13. The flow switch of any of the claims 1 to 12, wherein the plug (222, 422) comprises:
a spherical plug (222), or
a hemispherical plug (422), particularly, wherein the plug further comprises an annular structure disposed radially outward from, and mechanically coupled to, the hemispherical plug, wherein the annular structure defines proximal and distal grooves configured to engage with proximal and distal springs, respectively.

14. The flow switch of any of the claims 1 to 13, wherein the housing (212, 412) comprises a proximal housing portion defining the proximal opening (220A, 420A) and a distal housing portion defining the distal opening, and wherein the flow switch further comprises an O-ring configured to fluidically seal the proximal housing portion to the distal housing portion, or wherein flow switch further comprises a bypass mechanism configured to prevent the plug from moving proximally when the fluid flow is present within the internal cavity.

15. A medical aspiration system comprising:
a suction source (102);
aspiration tubing (116) fluidically coupled to the suction source (102), wherein the aspiration tubing (116) defines an inner lumen; and
a flow switch of any of the preceding claims, wherein the flow switch is fluidically coupled to the aspiration tubing (116).

## Patentansprüche

1. Strömungsschalter umfassend:
ein Gehäuse (212, 412), das einen inneren Hohlraum (214, 414) und proximale und distale Öffnungen (220A, 220B) zu dem inneren Hohlraum (214, 414) definiert, wobei der innere Hohlraum (214, 414) dazu ausgelegt ist, einen angesaugten Fluidstrom von einem Katheter aufzunehmen; und
einen Stopfen (222, 422), der in dem inneren Hohlraum (214, 414) angeordnet ist, wobei der Stopfen (222, 422) dazu ausgelegt ist:
sich als Reaktion auf eine über einem Schwellenwert liegende Schleppkraft des Fluidstroms, die auf eine distal zeigende Fläche (222B) des Stopfens (222, 422) ausgeübt wird, proximal zu bewegen, um die proximale Öffnung (220A, 420A) zu schließen; und
sich als Reaktion auf Fehlen des Fluidstroms in dem inneren Hohlraum (214, 414) distal zu bewegen, um die proximale Öffnung (220A, 420A) zu öffnen.

2. Strömungsschalter nach Anspruch 1, ferner umfassend:
eine distale Feder (224B, 424B), die zwischen der distal zeigenden Fläche des Stopfens und einer Innenfläche des Gehäuses (212, 412) angeordnet ist; und
eine proximale Feder (224A, 424A), die zwischen einer proximal zeigenden Fläche des Stopfens (222, 422) und der Innenfläche des Gehäuses (212, 412) angeordnet ist, wobei die proximale und die distale Feder dazu ausgelegt sind, den Stopfen (222, 422) ohne Vorhandensein des angesaugten Fluidstroms in dem inneren Hohlraum distal weg von der proximalen Öffnung (220A, 420A) vorzuspannen.

3. Strömungsschalter nach Anspruch 2, ferner umfassend einen Eingabemechanismus, dazu ausgelegt, eine Druck-oder Ausdehnungskraft, die durch die proximale Feder oder die distale Feder auf den Stopfen (222, 422) ausgeübt wird, zu modifizieren, um eine Größe der Schleppkraft zu modifizieren, die erforderlich ist, um den Stopfen (222, 422) proximal zu bewegen, um die proximale Öffnung (220A, 420A) zu schließen.

4. Strömungsschalter nach Anspruch 3, wobei der Eingabemechanismus ein drehbares Gewindeelement umfasst.

5. Strömungsschalter nach einem der Ansprüche 2 bis 4, wobei der innere Hohlraum einen Fluidströmungsweg und einen von dem Fluidströmungsweg radial nach außen gerichteten Ringraum definiert, wobei die proximale und die distale Feder innerhalb des Ringraums angeordnet sind.

6. Strömungsschalter nach einem der Ansprüche 1 bis 5, wobei das Gehäuse (212, 412) ferner mehrere Auslaufkanäle definiert, die von einem distalen Hohlraumabschnitt des inneren Hohlraums distal zu dem Stopfen (222, 422) zu einem proximalen Hohlraumabschnitt des inneren Hohlraums proximal zu dem abgerundeten Stopfen (222, 422) verlaufen, wobei die mehreren Auslaufkanäle dazu ausgelegt sind, eine Fluiddruckdifferenz zwischen dem proximalen Hohlraumabschnitt und dem distalen Hohlraumabschnitt zu modulieren.

7. Strömungsschalter nach Anspruch 6, ferner umfassend einen Auswahlschalter, dazu ausgelegt, verschiedene Auslaufkanäle der mehreren Auslaufkanäle zu öffnen oder zu schließen, um eine Zeitverzögerung zwischen Entfernen des Fluidstroms in dem inneren Hohlraum und Wiederöffnen der proximalen Öffnung (220A, 420A) durch den Stopfen (222, 422) zu modulieren.

8. Strömungsschalter nach einem der Ansprüche 6 oder 7, ferner umfassend einen Eingabemechanismus, dazu ausgelegt, wenigstens einen Auslaufkanal der mehreren Auslaufkanäle zu öffnen oder zu verschließen, um eine Zeitverzögerung zwischen Entfernen des Fluidstroms in dem inneren Hohlraum und Wiederöffnen der proximalen Öffnung (220A, 420A) durch den Stopfen (222, 422) zu modulieren.

9. Strömungsschalter nach Anspruch 8, wobei der Eingabemechanismus ein einstellbares Nadelventil und eine externe Einstellschraube umfasst.

10. Strömungsschalter nach einem der Ansprüche 6 bis 9, wobei die Fluiddruckdifferenz dazu ausgelegt ist, den Strömungsschalter in einer geschlossenen Konfiguration zu halten, indem der Stopfen (222, 422) wenigstens proximal über der proximalen Öffnung (220A, 420A) gehalten wird.

11. Strömungsschalter nach einem der Ansprüche 6 bis 10, wobei die Auslaufkanäle der mehreren Auslaufkanäle umfangsseitig um eine zentrale Längsachse des Strömungsschalters verteilt sind.

12. Strömungsschalter nach einem der Ansprüche 1 bis 11, wobei das Gehäuse (212, 412) dazu ausgelegt ist, fluidisch an einen proximalen Aspirationsschlauch an der proximalen Öffnung (220A, 420A) gekoppelt zu werden und fluidisch an einen langgestreckten Körper des Katheters an der distalen Öffnung gekoppelt zu werden.

13. Strömungsschalter nach einem der Ansprüche 1 bis 12, wobei der Stopfen (222, 422) umfasst:
einen kugelförmigen Stopfen (222) oder
einen halbkugelförmigen Stopfen (422), insbesondere wobei der Stopfen ferner eine ringförmige Struktur umfasst, die radial außerhalb des halbkugelförmigen Stopfens angeordnet und mechanisch daran gekoppelt ist, wobei die ringförmige Struktur proximale und distale Nuten definiert, die dazu ausgelegt sind, mit proximalen bzw. distalen Federn in Eingriff zu kommen.

14. Strömungsschalter nach einem der Ansprüche 1 bis 13, wobei das Gehäuse (212, 412) einen proximalen Gehäuseabschnitt, der die proximale Öffnung (220A, 420A) definiert, und einen distalen Gehäuseabschnitt, der die distale Öffnung definiert, umfasst und wobei der Strömungsschalter ferner einen O-Ring umfasst, der dazu ausgelegt ist, den proximalen Gehäuseabschnitt fluidisch gegen den distalen Gehäuseabschnitt abzudichten, oder wobei der Strömungsschalter ferner einen Umgehungsmechanismus umfasst, der dazu ausgelegt ist, zu verhindern, dass sich der Stopfen proximal bewegt, wenn der Fluidstrom in dem inneren Hohlraum vorhanden ist.

15. Medizinisches Aspirationssystem umfassend:
eine Ansaugquelle (102);
einen Aspirationsschlauch (116), der fluidisch an die Ansaugquelle (102) gekoppelt ist, wobei der Aspirationsschlauch (116) ein inneres Lumen definiert; und
einen Strömungsschalter nach einem der vorstehenden Ansprüche, wobei der Strömungsschalter fluidisch an den Aspirationsschlauch (116) gekoppelt ist.

## Revendications

1. Interrupteur d'écoulement comprenant :
un boîtier (212, 412) définissant une cavité interne (214, 414) et des ouvertures proximale et distale (220A, 220B) vers la cavité interne (214, 414), dans lequel la cavité interne (214, 414) est configurée pour recevoir un écoulement de fluide aspiré provenant d'un cathéter ; et
un bouchon (222, 422) disposé à l'intérieur de la cavité interne (214, 414), dans lequel le bouchon (222, 422) est configuré pour :
se déplacer proximalement, en réponse à une force de traînée supérieure à un seuil exercée par l'écoulement de fluide et appliquée à une surface orientée distalement (222B) du bouchon (222, 422), pour fermer l'ouverture proximale (220A, 420A) ; et
se déplacer distalement pour ouvrir l'ouverture proximale (220A, 420A) en réponse à une absence de l'écoulement de fluide à l'intérieur de la cavité interne (214, 414).

2. Interrupteur d'écoulement selon la revendication 1, comprenant en outre :
un ressort distal (224B, 424B) positionné entre la surface orientée distalement du bouchon et une surface intérieure du boîtier (212, 412) ; et
un ressort proximal (224A, 424A) positionné entre une surface orientée proximalement du bouchon (222, 422) et la surface intérieure du boîtier (212, 412), dans lequel les ressorts proximal et distal sont configurés pour solliciter le bouchon (222, 422) distalement à l'écart de l'ouverture proximale (220A, 420A) en l'absence de l'écoulement de fluide aspiré à l'intérieur de la cavité interne.

3. Interrupteur d'écoulement selon la revendication 2, comprenant en outre un mécanisme d'entrée configuré pour modifier une force de compression ou d'expansion appliquée par le ressort proximal ou le ressort distal au bouchon (222, 422) pour modifier une amplitude de la force de traînée requise pour déplacer le bouchon (222, 422) proximalement pour fermer l'ouverture proximale (220A, 420A).

4. Interrupteur d'écoulement selon la revendication 3, dans lequel le mécanisme d'entrée comprend un élément rotatif fileté.

5. Interrupteur d'écoulement selon l'une quelconque des revendications 2 à 4, dans lequel la cavité interne définit un trajet d'écoulement de fluide et un espace annulaire orienté radialement vers l'extérieur à partir du trajet d'écoulement de fluide, dans lequel les ressorts proximal et distal sont disposés à l'intérieur de l'espace annulaire.

6. Interrupteur d'écoulement selon l'une quelconque des revendications 1 à 5, dans lequel le boîtier (212, 412) définit en outre une pluralité de canaux de fuite s'étendant d'une partie de cavité distale de la cavité interne distale par rapport au bouchon (222, 422), à une partie de cavité proximale de la cavité interne proximale par rapport au bouchon arrondi (222, 422), dans lequel la pluralité de canaux de fuite est configurée pour moduler un différentiel de pression de fluide entre la partie de cavité proximale et la partie de cavité distale.

7. Interrupteur d'écoulement selon la revendication 6, comprenant en outre un interrupteur de sélection configuré pour ouvrir ou fermer différents canaux de fuite de la pluralité de canaux de fuite afin de moduler un retard entre un retrait de l'écoulement de fluide à l'intérieur de la cavité interne et une réouverture de l'ouverture proximale (220A, 420A) par le bouchon (222, 422).

8. Interrupteur d'écoulement selon l'une quelconque des revendications 6 ou 7, comprenant en outre un mécanisme d'entrée configuré pour ouvrir ou obturer au moins un canal de fuite de la pluralité de canaux de fuite afin de moduler un retard entre un retrait de l'écoulement de fluide à l'intérieur de la cavité interne et une réouverture de l'ouverture proximale (220A, 420A) par le bouchon (222, 422).

9. Interrupteur de débit selon la revendication 8, dans lequel le mécanisme d'entrée comprend une vanne à pointeau réglable et une vis de réglage externe.

10. Interrupteur d'écoulement selon l'une quelconque des revendications 6 à 9, dans lequel le différentiel de pression de fluide est configuré pour retenir l'interrupteur d'écoulement dans une configuration fermée au moins en maintenant le bouchon (222, 422) de manière proximale sur l'ouverture proximale (220A, 420A).

11. Interrupteur d'écoulement selon l'une quelconque des revendications 6 à 10, dans lequel les canaux de fuite de la pluralité de canaux de fuite sont répartis circonférentiellement autour d'un axe longitudinal central de l'interrupteur d'écoulement.

12. Interrupteur d'écoulement selon l'une quelconque des revendications 1 à 11, dans lequel le boîtier (212, 412) est configuré pour s'accoupler fluidiquement à un tube d'aspiration proximal au niveau de l'ouverture proximale (220A, 420A), et pour s'accoupler fluidiquement à un corps allongé du cathéter au niveau de l'ouverture distale.

13. Interrupteur d'écoulement selon l'une quelconque des revendications 1 à 12, dans lequel le bouchon (222, 422) comprend :
un bouchon sphérique (222), ou
un bouchon hémisphérique (422), en particulier, dans lequel le bouchon comprend en outre une structure annulaire disposée radialement vers l'extérieur à partir du bouchon hémisphérique, et accouplée mécaniquement à celui-ci, dans lequel la structure annulaire définit des rainures proximale et distale configurées pour venir en prise avec des ressorts proximal et distal, respectivement.

14. Interrupteur d'écoulement selon l'une quelconque des revendications 1 à 13, dans lequel le boîtier (212, 412) comprend une partie de boîtier proximale définissant l'ouverture proximale (220A, 420A) et une partie de boîtier distale définissant l'ouverture distale, et dans lequel l'interrupteur d'écoulement comprend en outre un joint torique configuré pour sceller fluidiquement la partie de boîtier proximale à la partie de boîtier distale, ou dans lequel l'interrupteur d'écoulement comprend en outre un mécanisme de dérivation configuré pour empêcher le bouchon de se déplacer de manière proximale lorsque l'écoulement de fluide est présent à l'intérieur de la cavité interne.

15. Système d'aspiration médical comprenant :
une source d'aspiration (102) ;
un tube d'aspiration (116) accouplé fluidiquement à la source d'aspiration (102), le tube d'aspiration (116) définissant une lumière interne ; et
un interrupteur d'écoulement selon l'une quelconque des revendications précédentes, l'interrupteur d'écoulement étant accouplé fluidiquement au tube d'aspiration (116).
